# EUROPEAN PATENT SPECIFICATION

(11) **EP 1 550 671 B1**
(45) Date of publication and mention of the grant of the patent: **14.03.2007**
(21) Application number: 04000067.1
(22) Date of filing: 05.01.2004
(51) Int. Cl.: C07K 14/47

(54) **Placenta derived apoptotic factor**
Apoptotischer Faktor aus Plazenta
Facteur apoptotique dérivé du placenta

(43) Date of publication of application: 06.07.2005
(73) Proprietor: Anawrahta Biotech Co. Ltd., Tanshui Town, Taipeh Shien (TW)
(72) Inventor: Wei-Yu, Lo, Tanshui Town Taipeh Shien (TW); Shie-Liang, Hsieh, Tanshui Town Taipeh Shien (TW)
(74) Representative: Becker Kurig Straus

(56) References cited:
- WO-A-99/57270
- WICHEREK ET AL: "Comparative analysis of RCAS1 level in neoplasms and placenta" ACTA BIOCHIMICA POLONICA, vol. 50, 2003, pages 1187-1194, XP002288295

### SEQUENCE LISTING

<110> Wei-Yu LO
   Shie-Liang HSIEH
<120> Placenta Derived Apoptotic Factor and Its Gene
<130> 6653-015
<140> 09/684,327
   <141> 2000-10-10
<160> 9
<170> FastSEQ for Windows Version 4.0
<210> 1
   <211> 258
   <212> PRT
   <213> PDAF Polypeptide Sequence
<400> 1
   Met Ala Ile Thr Gln Phe Arg Leu Phe Lys Phe Cys Thr Cys Leu Ala
    1               5                  10                  15
   Thr Val Phe Ser Phe Leu Lys Arg Leu Ile Cys Arg Ser Gly Arg Gly
               20                  25                  30
   Arg Lys Leu Ser Gly Asp Gln Ile Thr Leu Pro Thr Thr Val Asp Tyr
           35                  40                  45
   Ser Ser Val Pro Lys Gln Thr Asp Val Glu Glu Trp Thr Ser Trp Asp
       50                  55                  60
   Glu Asp Ala Pro Thr Ser Val Lys Ile Glu Gly Gly Asn Gly Asn Val
   65                  70                  75                  80
   Ala Thr Gln Gln Asn Ser Leu Glu Gln Leu Glu Pro Asp Tyr Phe Lys
                   85                  90                  95
   Asp Met Thr Pro Thr Ile Arg Lys Thr Gln Lys Ile Val Ile Lys Lys
               100                 105                 110
   Arg Glu Pro Leu Asn Phe Gly Ile Pro Asp Gly Ser Thr Gly Phe Ser
           115                 120                 125
   Ser Arg Leu Ala Ala Thr Gln Asp Leu Pro Phe Ile His Gln Ser Ser
       130                 135                 140
   Glu Leu Gly Asp Leu Asp Thr Trp Gln Glu Asn Thr Asn Ala Trp Glu
   145                 150                 155                 160
   Glu Glu Glu Asp Ala Ala Trp Gln Ala Glu Glu Val Leu Arg Ser Arg
                   165                 170                 175
   Thr Asn Val Cys Leu Leu Cys Ser Leu Leu Phe His His Pro Thr Pro
               180                 185                 190
   Thr Ser Thr Pro Tyr Ile Asn Gln Ser Val Lys Ile Glu Arg Val Ser
           195                 200                 205
   Leu Gly Gln Trp Ser Tyr Gly Lys Ser Lys Glu Gln Gln Lys Leu Ala
       210                 215                 220
   Asp Arg Glu Lys Arg Ala Ala Glu Gln Gln Arg Lys Lys Met Glu Lys
   225                 230                 235                 240
   Glu Ala Gln Arg Leu Met Lys Lys Glu Gln Asn Lys Ile Gly Val Lys
                   245                 250                 255
   Leu Ser
<210> 2
   <211> 45
   <212> PRT
   <213> PDAF Polypeptide Sequence
<400> 2
   Ser Arg Thr Asn Val Cys Leu Leu Cys Ser Leu Leu Phe His His Pro
    1               5                  10                  15
   Thr Pro Thr Ser Thr Pro Tyr Ile Asn Gln Ser Val Lys Ile Glu Arg
               20                  25                  30
   Val Ser Leu Gly Gln Trp Ser Tyr Gly Lys Ser Lys Glu
           35                  40                  45
<210> 3
   <211> 84
   <212> PRT
   <213> PDAF Polypeptide Sequence
<400> 3
   Ser Arg Thr Asn Val Cys Leu Leu Cys Ser Leu Leu Phe His His Pro
    1              5                   10                  15
   Thr Pro Thr Ser Thr Pro Tyr Ile Asn Gln Ser Val Lys Ile Glu Arg
               20                  25                  30
   Val Ser Leu Gly Gln Trp Ser Tyr Gly Lys Ser Lys Glu Gln Gln Lys
           35                  40                  45
   Leu Ala Asp Arg Glu Lys Arg Ala Ala Glu Gln Gln Arg Lys Lys Met
       50                  55                  60
   Glu Lys Glu Ala Gln Arg Leu Met Lys Lys Glu Gln Asn Lys Ile Gly
   65                  70                  75                  80
   Val Lys Leu Ser
<210> 4
   <211> 774
   <212> DNA
   <213> PDAF Polypeptide Sequence
<400> 4
   atggccatca cccagtttcg gttatttaaa ttttgtacct gcctagcaac agtattctca    60
   ttcctaaaga gattaatatg cagatctggc agaggacgga aattaagtgg agaccaaata   120
   actttgccaa ctacagttga ttattcatca gttcctaagc agacagatgt tgaagagtgg   180
   acttcctggg atgaagatgc acccaccagt gtaaagatcg aaggagggaa tgggaatgtg   240
   gcaacacaac aaaattcttt ggaacaactg gaacctgact attttaagga catgacacca   300
   actattagga aaactcagaa aattgttatt aagaagagag aaccattgaa ttttggcatc   360
   ccagatggga gcacaggttt ctctagtaga ttagcagcta cacaagatct gccttttatt   420
   catcagtctt ctgaattagg tgacttagat acctggcagg aaaataccaa tgcatgggaa   480
   gaagaagaag atgcagcctg gcaagcagaa gaagttctga gatccaggac caatgtatgt   540
   ttactctgct ctctcctgtt tcatcatccc actcctacct ccactcccta cattaaccaa   600
   tcagtaaaga tagagagagt gagtctgggt cagtggagtt acggaaagag taaggaacag   660
   cagaaactag cagacagaga aaagagagca gccgaacaac aaaggaagaa aatggaaaag   720
   gaagcacaac ggctaatgaa gaaggaacaa aacaaaattg gtgtgaaact ttca         774
<210> 5
   <211> 135
   <212> DNA
   <213> PDAF Polypeptide Sequence
<400> 5
   tccaggacca atgtatgttt actctgctct ctcctgtttc atcatcccac tcctacctcc    60
   actccctaca ttaaccaatc agtaaagata gagagagtga gtctgggtca gtggagttac   120
   ggaaagagta aggaa                                                     135
<210> 6
   <211> 252
   <212> DNA
   <213> PDAF Polypeptide Sequence
<400> 6
   tccaggacca atgtatgttt actctgctct ctcctgtttc atcatcccac tcctacctcc    60
   actccctaca ttaaccaatc agtaaagata gagagagtga gtctgggtca gtggagttac   120
   ggaaagagta aggaacagca gaaactagca gacagagaaa agagagcagc cgaacaacaa   180
   aggaagaaaa tggaaaagga agcacaacgg ctaatgaaga aggaacaaaa caaaattggt   240
   gtgaaacttt ca                                                        252
<210> 7
   <211> 8
   <212> PRT
   <213> PDAF Polypeptide Sequence
<220>
   <221> VARIANT
   <222> (1)...(8)
   <223> Xaa = Any Amino Acid
<400> 7
   Gly Xaa Xaa Xaa Xaa Gly Lys Ser
    1               5
<210> 8
   <211> 6
   <212> PRT
   <213> PDAF Polypeptide Sequence
<220>
   <221> VARIANT
   <222> (1)...(6)
   <223> Xaa = Any Amino Acid
<400> 8
   Xaa Xaa Xaa Xaa Gly Lys
    1               5
<210> 9
   <211> 29
   <212> PRT
   <213> PIG-B Polypeptide Sequence
<400> 9
   Leu Ile Leu His His Phe Leu Pro Val Gly Phe Val Thr Leu Ser
    1               5                  10                  15
   Leu Ser Leu Met Ile Asp Arg Ile Phe Phe Gly Gln Trp Thr
               20                  25

## Description

### Field of the Invention

The present invention relates to a novel human tumor-associated antigen, designated as placenta derived apoptotic factor (PDAF), and the genes thereof.

### Description of the Prior Art

A number of cancer-specific genetic markers and tumor-associated antigens have been identified. It is found that tumor-associated antigens are mostly surface molecules that are expressed in tumor cells, instead of the cells of non-tumor tissues. Tumor-associated antigens have been characterized either as membrane proteins or altered carbohydrate molecules of glycoproteins and glycolipids. They make tumor cells immunologically distinct from normal cells, which are useful as diagnostic and therapeutic targets for human cancers.

Some tumor-associated antigens have been disclosed in prior references. For instance, a receptor-binding cancer antigen expressed on SiSo cells (RCAS1) functioning as a tumor-associated antigen, has been described by Takeshi Watanabe et al., 1999, Nature Medicine, Vol. 5, No. 8, pp.938-942. RCAS1 gene does not express in normal cells, but expresses in uterine cervical adenocarcinoma, uterine endomertrial adenocarcinoma, ovarian carcinoma and uterine cervical squamous cell carcinoma (see, Takeshi Watanabe et al., 1996, American Cancer Society, pp.1501-1509; and Hitoo Nakano, 1998, Clinical Cancer Research, vol. 4, pp. 1517-1520). In addition, the RCAS1 gene can also be expressed in esophageal squamous cell carcinoma, gastric adenocarcinoma, colon adenocarcinoma and pancreatic adenocarcinoma. RCAS1 is a protein comprising 213 amino acids. Such protein has an N-terminal transmembrane segments (8-27 amino acids) and a coiled-coil structure in the C-terminal portion (179-206 amino acids), indicating that RCAS1 may be a type II membrane protein or a secreted protein having a structure of dimeric protein. RCAS 1 acts as a ligand for a putative receptor present on various human cell lines such as K562 (human chronic myelogeneous leukemia), CCRF-CEM (human T lymphoblast) and Ramos (Burkitt lymphoma) and normal peripheral lymphcytes such as T, B and NK cells. RCAS1 inhibits the growth of receptor-expressing cells and induces apoptotic cell death. Tumor cells may evade immune surveillance by expressing RCAS1 and induce apoptosis in RCAS1 receptor-positive immune cells. RCAS1 plays roles in both tumor-associated antigen and induction of apoptosis of the immune cells. An analysis of RCAS1 level in neoplasm and placenta was described by Wicherek et al, Acta Biochimica Polonica, Vol 50, 2003, PP-1187-1194.

It is expected that new tumor-associated antigens, similar to the tumor-associated RCAS1 antigen and the nucleic acids coding for the antigens, are useful in diagnosing, preventing, and treating immune disorders, cell proliferation and tumors, particularly cancers.

### SUMMARY OF THE INVENTION

One object of the invention is to provide a novel human tumor-associated antigen, designated as placenta derived apoptotic factor (PDAF), which comprises an amino acid sequence of SEQ ID NO:1.

One object of the invention is to provide a fragment of the PDAF, comprising an amino acid sequence of SEQ ID NO:2 or a sequence which is 70% identical and retains the properties of the former.

One object of the invention is to provide a fragment of the PDAF, comprising an amino acid sequence of SEQ ID NO:3 or a sequence which is 70% identical and retains the properties of the former.

Another object of the invention is to provide the nucleic acid of SEQ ID NO: 4 encoding the polypeptide of SEQ ID NO: 1 and the degenerate sequences thereof. Also, the invention provides the nucleic acid of SEQ ID NO: 5 encoding the polypeptide of SEQ ID NO: 2 and the degenerate sequences thereof; and the nucleic acid of SEQ ID NO: 6 encoding the polypeptide of SEQ ID NO: 3 and the degenerate sequences thereof.

Another object of the invention is to provide an expression vector containing the nucleic acid of SEQ ID NO: 4, an expression vector containing the nucleic acid of SEQ ID NO: 5, and an expression vector containing the nucleic acid of SEQ ID NO: 6. In addition, the invention provides a host cell containing the nucleic acid of SEQ ID NO: 4, a host cell containg the nucleic acid of SEQ ID NO: 5, and a host cell containing the nucleic acid of SEQ ID NO: 6.

Another object of the invention is to provide a pharmaceutical composition comprising the PDAF of the invention, or the fragments thereof.

Another further object of the invention is to provide transgenic animals, which is introduced with a gene fragment containing the nucleic acid sequence of SEQ ID NO: 4, or a gene fragment containing the nucleic acid sequence of SEQ ID NO: 5, or a gene fragment containing the nucleic acid sequence of SEQ ID NO: 6.

### BRIEF DESCRIPTION OF THE DRAWINGS

Figs. 1A-1D shows the amino acid sequence of PDAF (SEQ ID NO.1) and the coding nucleic acid sequence thereof (SEQ ID NO.4). The box region is a novel peptide consists of 45 amino acids in the PDAF protein; the shadow region is a ATP-GTP binding site motif; and in the underline region, TM represents a transmembrane domain, and DIM represents a coiled coil structure and dimeric domain.
Fig. 2A shows the restriction maps of RCAS1 and PDAF genes.
Fig, 2B shows the electrophoresis result of RCAS1 and PDAF genes treated with certain restriction enzymes. Lines 1 and 5 are untreated samples; lines 2 and 6 are EcoRI treated samples; lines 3 and 7 are Bgl II treated samples; lines 4 and 8 are Apol I treated samples; M1 is GeneRuler^{™} 1kb DNA Ladder; and M2 is GeneRuler^{™} 100 bp DNA Ladder Plus.

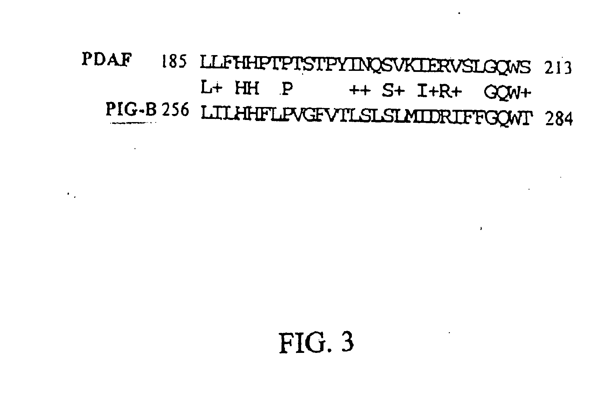

Fig. 3 shows the similarity between the amino acids 185-213 of PDAF (SEQ ID NO.1) and the amino acids 256-284 of PIG-B (SEQ ID NO.9). The identity is 34% (10/29) and the positive rate is 58% (17/29).

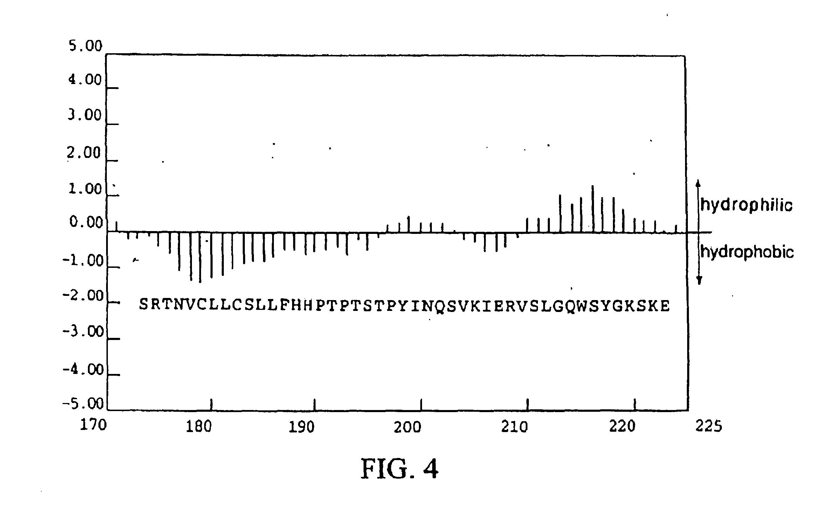

Fig. 4 shows hydropathy analysis of PDAF.

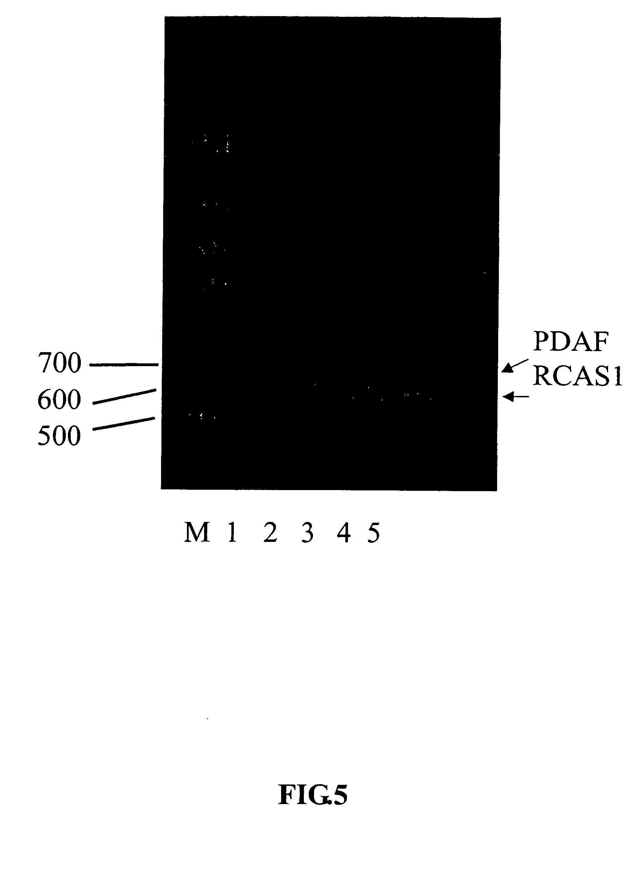

Fig. 5 shows the expression of RCAS1 and PDAF in various human tumor cell lines. M represents 100 bp DNA marker; and lines 1 to 5 represents MCF 7 cell line, HT29 cell line, RD cell line, A375 cell line and placenta, respectively.

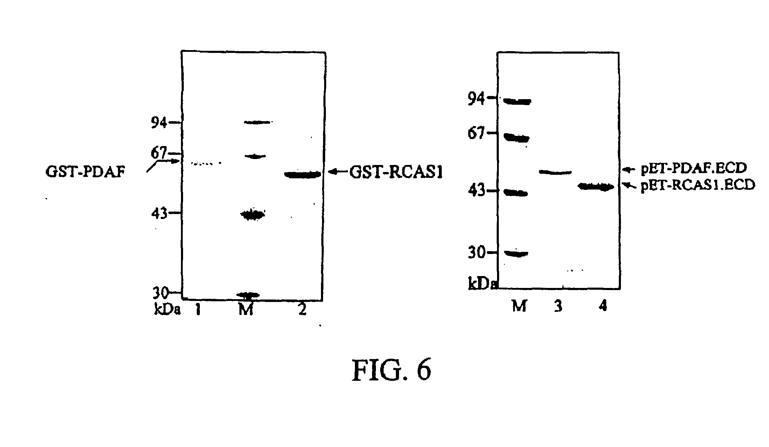

Fig. 6 shows the expressions of recombinant fusion proteins GST-RCAS1 GST-PDAF, pET-PDAF.ECD and pET-RCAS1.ECD

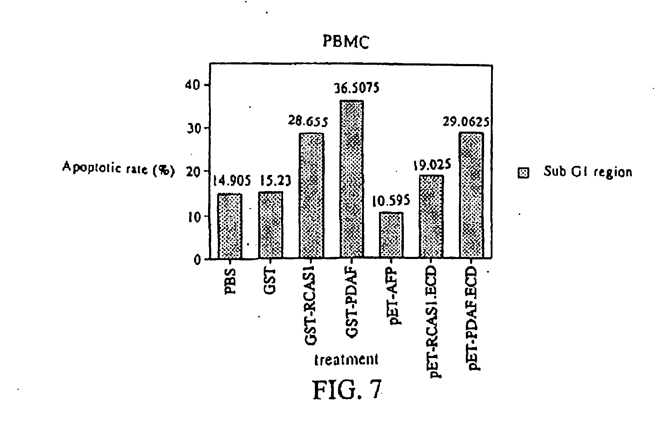

Fig. 7 shows the apoptotic rate of PBMC cell line after the treatment of RCAS1, PDAF, RCAS1.ECD, and PDAF.ECD.

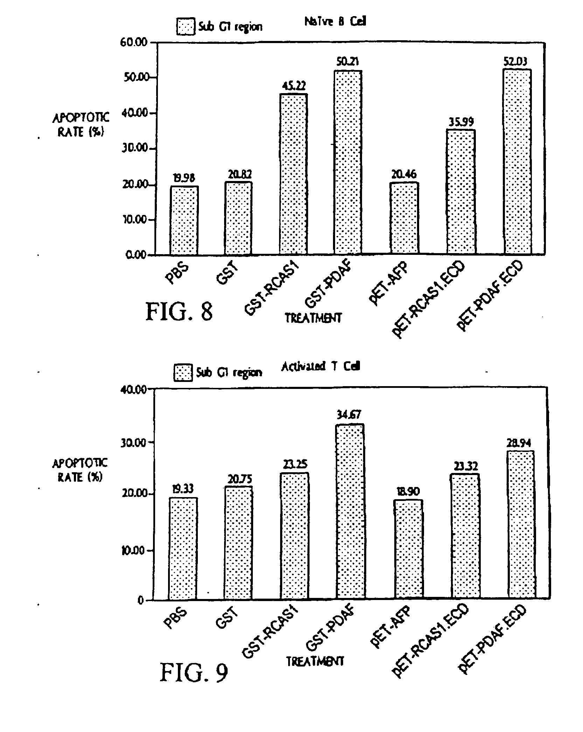

Fig. 8 shows the apoptotic rate of native B cell line after the treatment of RCAS1, PDAF, RCAS1.ECD, and PDAF.ECD.
Fig. 9 shows the apoptotic rate of T cell line after the treatment of the RCAS1, PDAF, RCAS1.ECD, and PDAF.ECD.

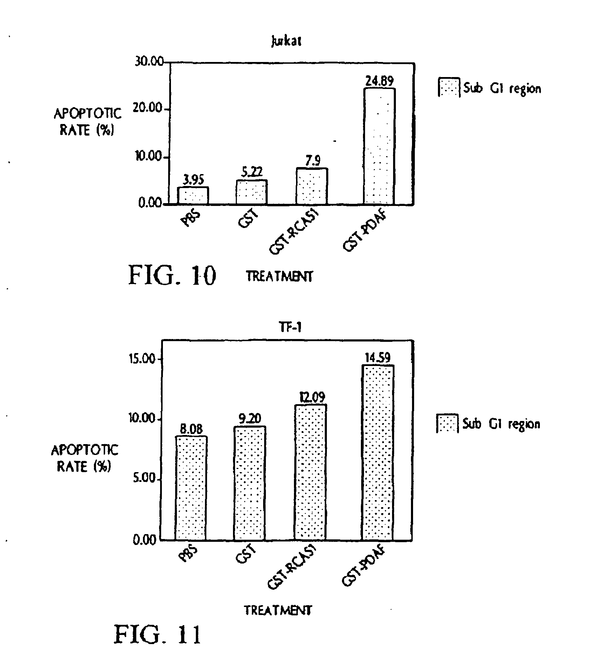

Fig. 10 shows the apoptotic rate of Jurkat cell line after the treatment of the RCAS1 and PDAF.
Fig. 11 shows the apoptotic rate of TF-1 cell line after the treatment of the RCAS1 and PDAF.

### DETAILED DESCRIPTION OF THE INVENTION

The present invention features a novel tumor-associated antigen hereinafter designated PDAF and characterized by having a similarity to RCAS 1. It is surprisingly found that as compared with RCAS 1, the PDAF of the invention shows a higher apoptotic rate to the tumor cells and immune cells.

### Definitions

The term "nucleic acid sequence", as used herein, refers to peptide nucleic acid (PNA; The FASEB Journal, 2000, 14: (1041-1060)) and polynucleotides such as deoxyribonucleic acid (DNA), and, where appropriate, ribonucleic acid (RNA). The term should also be understood to include, as equivalents, analogs of either RNA or DNA made from nucleotide analogs, and, as applicable to the embodiment being described, single (sense or antisense) and double-stranded polynucleotides.

The term "amino acid sequence", as used herein, refers to an amino acid sequence of a naturally occurring protein molecule, "amino acid sequence" and like terms, such as "polypeptide" or "protein" are not meant to limit the amino acid sequence to the complete, native amino acid sequence associated with the recited protein molecule. Amino acid sequence includes an oligopeptide, peptide, polypeptide, or protein sequence, and fragments or portions thereof, and to naturally occurring or synthetic molecules.

An amino acid sequence is altered by one or more amino acid substitutions, deletions, and/or additions. The amino acid sequence may have "conservative" changes, wherein a substituted amino acid has similar structural or chemical properties, e.g., replacement of leucine with isoleucine. More rarely, an amino acid sequence may have "nonconservative" changes, e.g., replacement of a glycine with a tryptophan. Similar minor variations may also include amino acid deletions or insertions, or both.

The term "deletion", as used herein, refers to a change in either amino acid or nucleotide sequence in which one or more amino acid or nucleotide residues, respectively, are absent.

The term "insertion" or "addition", as used herein, refers to a change in an amino acid or nucleotide sequence resulting in the addition of one or more amino acid or nucleotide residues, respectively, as compared to the naturally occurring molecule.

The term "derivative", as used herein, refers to the chemical modification of a nucleic acid. Illustration of such modifications would be replacement of hydrogen by an alkyl, acyl, or amino group. A nucleic acid derivative would encode a polypeptide which retains essential biological characteristics of the natural molecule.

The term "biologically function equivalent", as used herein, refers to a protein or polypeptide, wherein certain amino acid residues are deleted or substituted with other amino acid residues, or the protein or polypeptide sequence are added with other amino acid residues that do not substantially affect the function or activity (e.g., activities of treating a cell proliferation disorder or inhibiting an immune response, inhibiting the growth and differentiation of cells and treating a cancer) of the protein or polypeptide, even if in lesser or greater degree.

For instance, the polypeptide of the invention (i.e., SEQ ID NO: 2 or 3) may have some alterations in the amino acid sequence that is different from, but essentially identical to, the amino acid sequence of the polypeptide (i.e., SEQ ID NO: 1, 2 or 3), and has essentially identical properties of the polypeptide as described herein, even if in less or greater degree.

The term "the amino acid sequence is different from but essentially identical to the amino acid sequence of the polypeptide of SEQ ID NO : 2 or 3" or the like, as used herein, means that the amino acid sequence substantially corresponds to a portion of the amino acid sequence set forth in SEQ ID NO: 2 or 3 wherein relatively few amino acid residues are not identical to those at the corresponding positions of the amino acid sequence of SEQ ID NO: 2 or 3, but said sequence retains the properties of the polypeptide of SEQ ID NO: 1, 2 or 3 as described herein, even in less or greater degree.

Specifically, "the amino acid sequence being different from but essentially identical to the amino acid sequence of the polypeptide of SEQ ID NOS: 2 or 3" includes an amino acid sequence, in which 70% to about 80%, preferably about 81 to about 90%, more preferably about 91% to about 99% of the amino acid residues are identical or similar to those at the corresponding positions of the amino acid of SEQ ID NO: 2 or 3, and wherein a polypeptide or porotein having such amino acid sequence retains the properties of the polypeptide of SEQ ID NO: 2 or 3 as descried herein, even in less or greater degree.

The method for producing the polypeptide having essentially identical properties of a polypeptide or a protein, for instance, is to alter the amino acid sequences, said method being well known in the art such as genetic engineering techniques e.g., site-directed mutagenesis to modify the nucleotide acid sequences or the amino acid sequences and expression of recombinant proteins.

The term "fusion protein", as used herein, refers to a protein or a polypeptide consisting of a first amino acid sequence encoding a target protein or polypeptide and a second amino acid sequence encoding a different protein or polypeptide from the target protein or polypeptide, wherein the protein or polypeptide encoded by the second amino acid sequence, for instance, is glutathione-S-transferase, alkaline phosphatase or an epitope tag, which is provided for detection or purification of the target protein or polypeptide, for instance.

The term "vector", as used herein, refers to a nucleic acid molecule capable of transporting another nucleic acid to which it has been linked. One type of preferred vector is an episome, i.e., a nucleic acid capable of extra-chromosomal replication. Preferred vectors are those capable of autonomous replication and/expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer generally to circular double stranded DNA loops which, in their vector form are not bound to the chromosome.

The term "host cell", as used herein, refers to a cell of a host, which can be infected with a vector, such as a plasmid. The hosts suitable for the invention include those commonly and conventionally used in the art.

The term "transgenic animal ", as used herein, refers to any animal in which one or more, and preferably essentially all, of the cells of the animal introduced with a foreign gene. The gene is introduced into the animal cell, directly or indirectly by introduction into a precursor of the cell, by way of deliberate genetic manipulation, such as by microinjection or by infection with a recombinant vector. The term genetic manipulation includes not only classical cross-breeding, or in vitro fertilization, but also the introduction of a recombinant DNA molecule, which may be integrated within a chromosome or may be extrachromosomally replicating DNA.

### Polypeptides

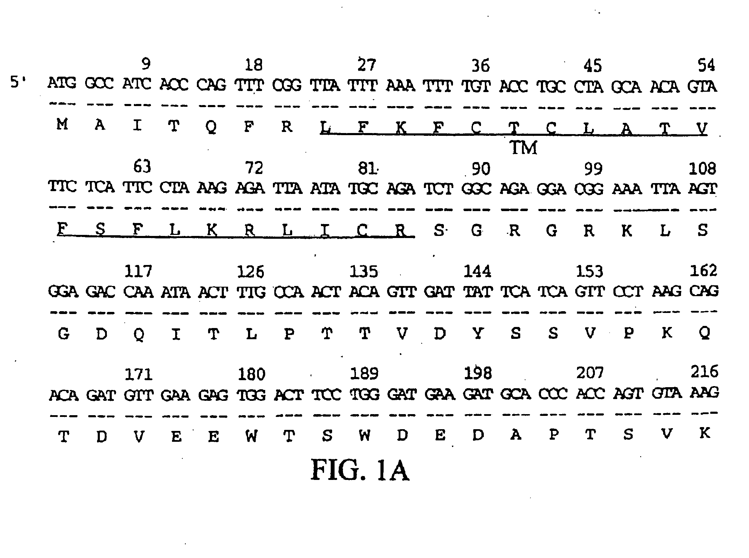

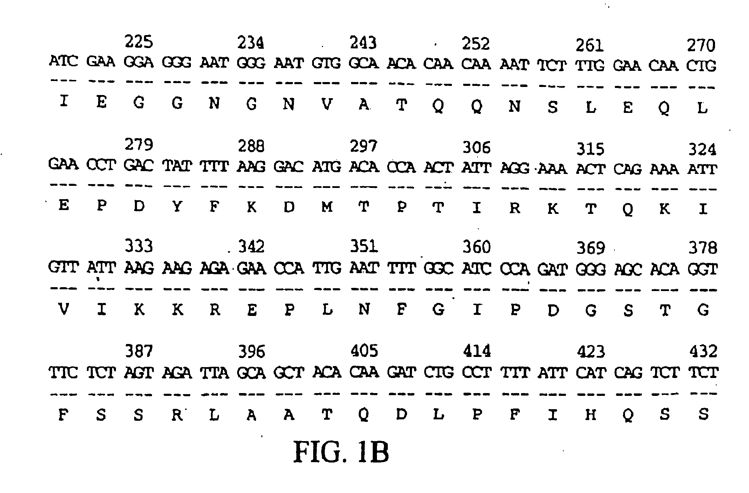

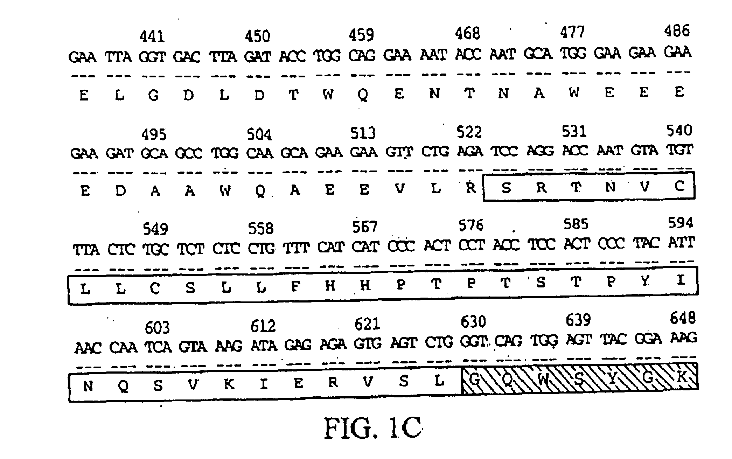

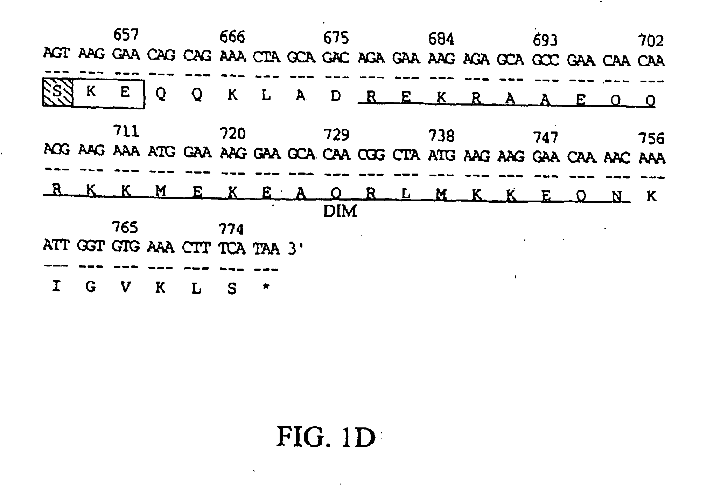

One object of the invention is to provide a polypeptide, designated as placenta derived apoptotic factor (PDAF), comprising the amino acid sequence as shown in SEQ ID NO:1, see Figs. 1A-1D.

The PDAF refers to the amino acid sequences of substantially purified PDAF obtained from any species, preferably human, from any source whether natural, synthetic, semi-synthetic, or recombinant.

According to the invention, PDAF comprises 258 amino acids, wherein 213 amino acids of PDAF are completely same as those of RCAS1. The sequence consisting of the 213 amino acids contains a transmembrane domain and a coiled-coil structure and dimeric domain. PDAF of the invention can induce apoptosis to the tumor cells and immune cells.

Another object of the invention is to provide a polypeptide consisting of 45 amino acids (SEQ ID NO: 2).

The 45 amino acids are at the positions 175-219 of PDAF (see Figs. 1 A-1D). As compared with the sequences of RCAS1, the 45 amino acids (SEQ ID NO.2) are inserted between amino acids 174 and 175 of RCAS1. Such sequence of 45 amino acids forms a hydrophobic region before the coiled-coil functional structure and dimeric domain of RCAS 1. Therefore, the hydrophobic region may affect the structure of the whole protein and thus the PDAF has an improved efficacy than RCAS1. In addition, it is found the amino acids at positions 210-217 in SEQ ID NO:2 are similar to the sequences of ATP-GTP binding site motif of Ras protein. The protooncogene ras is an essential gene for the growth and differentiation for various types of cells. Ras is a GTP binding protein, which controls the signal transduction by GTP hydrolysis. A single point mutation in the GTP binding motif of the ras gene accounts for more than 90% of all ras mutations and is present in more than 20% of all solid tumor. Therefore, mutant ras provides a potential target for cancer therapy (see, Journal of Immunotherapy, 1999, 22(2): 155-165 and The Journal of Urology, 1999, 162: 1519-1526). Given the above, we can reasonably expect that SEQ ID NO:2 encompassing the GTP-ATP binding motif is also important in the growth and differentiation of cells and can be used in the treatment and diagnosis of diseases.

In one embodiment of the invention, SEQ IN NO:2 is one having at least 80%, preferably 90%, and most preferably 95%, amino acid sequence identity or similarity to the SEQ ID NO:2.

One object of the invention is to provide a polypeptide sequence of consisting of the amino acids at positions 175-258 of PDAF (SEQ ID NO:3).

The polypeptide of SEQ ID NO:3 is a functional fragment mainly inducing the apoptosis for immune cells and tumor cells. In one embodiment of the invention, SEQ ID NO:3 is one having at least 80%, more preferably 90% and most preferably 95%, amino acid sequence identity or similarity to the SEQ ID NO:3.

### Nucleic Acids

Another object of the invention is to provide an isolated nucleic acid sequence (SEQ ID NO:4) and the altered sequences thereof, which encode PDAF (SEQ ID NO:1), as shown in Figs. 1A-1D. PDAF gene is similar to the RCAS1 gene, which consists of 774 DNAs in length. PDAF gene is cloned from human placenta and rhabdomyoma tumor RD cell line.

The invention also provides an isolated nucleic acid sequence (SEQ ID No: 5) consisting of 135 DNAs and the altered sequences thereof, which encodes the 45 amino acids as shown in SEQ ID NO: 2. The 135 DNAs sequences are inserted between positions 522 and 523 of the RCAS1 sequences. The numbers of such SEQ ID NO: 5 are a multiple of 3 and no translation stop codon exists in the region. Therefore, such a region does not affect the translation of the subsequent amino acid sequences and the length thereof.

The invention also provides an isolated nucleic acid sequence (SEQ ID No: 6) consisting of the nucleic acids at positions 523-774 of the PDAF DNAs and the altered sequences thereof, which encode the polypeptide of SEQ ID NO:3. Such sequence may be the functional fragments of PDAF.

The invention encompasses all nucleic acids which, by virtue of the well-known degeneracy of the genetic code, also encode the SEQ ID No: 1, 2 or 3 polypeptide. Such degenerate variants may be naturally-occurring or may be produced through routine application of molecular engineering techniques, e.g., Sambrook et al. (1989), Molecular Cloning: A Laboratory Manual, 2nd ed., Cold Spring Harbor Laboratory Press, Cold Spring Harbor, N.Y.

According to the invention, the production of DNA sequences, or fragments thereof, which encode the sequences SEQ ID Nos: 1, 2 and 3 and their derivatives, may use chemical methods well known in the art. Alternatively, they may be produced through chemical methods. For example, peptide synthesis can be performed using various solid-phase techniques and automated synthesis may be achieved through a peptide synthesizer, for example, ABI 431A Peptide Synthesizer.

Methods for DNA sequencing which are well known and generally available in the art may be used to practice any embodiments of the invention. The methods may employ such enzymes as the Klenow fragment of DNA polymerase I, Taq polymerase, T7 polymerase, or combinations of recombinant polymerases and proofreading exonucleases such as the ELONGASE or commercially available kits. Preferably, the process is automated with machines such as the Hamilton Micro Lab 2200 (Hamilton, Reno, Nev.), Peltier Thermal Cycler (PTC200; MJ Research, Watertown, Mass.) and the ABI 377 DNA sequencers (Perkin Elmer).

The nucleic acid sequences of the present invention can be engineered using methods generally known in the art in order to alter PDAF encoding sequences for a variety, including but not limited to, alterations which modify the cloning, processing, and/or expression of the gene product.

### Expression Vector and Host System

Another object of the invention is to provide an expression vector, containing the nucleic acid sequences as shown in SEQ ID No. 4, 5 or 6. In order to express a biologically active PDAF, the nucleic acid sequences encoding PDAF or functional equivalents, may be inserted into appropriate expression vector, i.e., a vector which contains the necessary elements for the transcription and translation of the inserted coding sequence. According to the invention, methods being well known to those skilled in the art may be used to construct expression vectors containing sequences encoding PDAF and appropriate transcriptional and translational control elements. These methods include in vitro recombinant DNA techniques, synthetic techniques, and in vivo genetic recombination.

Another object of the invention is to provide a host cell containing the expression vector containing the nucleic acid sequence as shown in SEQ ID No. 4, 5 or 6. According to the invention, a number of host systems may be utilized to contain and express sequences encoding PDAF. These include, but are not limited to, microorganisms such as bacteria transformed with recombinant bacteriophage, plasmid, or cosmid DNA expression vectors; yeast transformed with yeast expression vectors; insect cell systems infected with virus expression vectors; plant cell systems transformed with virus expression vectors or with bacterial expression vectors; or animal cell systems.

### Utility

According to the invention, it is surprisingly found that PDAF possesses the features as follows:
(1) As compared with RCAS1, the PDAF induces a higher apoptotic rate to the peripheral blood lymphocyte, native B cell, activated T cell and leukemia cell lines such as Jurkat and TF-1. Therefore, PDAF can be used as a cytotoxic agent for the treatment of leukemia, and/or an immunosuppressive agent for the disorders associated with the need of inhibiting response such as graft versus host diseases, autoimmune diseases and course of transplantation.
(2) In addition, the expression of PDAF is higher than that of RCAS1 in placenta tissue. Moreover, PDAF can induce the apoptosis of the immune cells. Based on the teachings of David A. Clark, 1991, Critical Reviews in Immunology, 11 (3,4): 215-247, we can reasonably respect that PDAF can be used in reducing sterility caused by the rejection to sperm and abortion caused by the rejection to fetus tissue.
(3) PDAF can be expressed in the tumor cells. Therefore, we can expect that the antibodies against PDAF can be used in killing the tumor cells expressing RCAS1 or PDAF.
(4) The expression of PDAF is lower than that of RCAS1 in some tumor cells. Therefore, the expression of the PDAF has higher tissue specificity than that of RCAS1. We can thus expect that PDAF can be used in the diagnosis of cancer.
(5) It is known in the art that immunosuppressive agent can be used in the treatment of hepatitis (see, Crit Rev Immunol, 1991, 11 (3-4):215-247). Therefore, we can reasonably expect that the PDAF of the invention may be used in treating hepatitis.

Given the above, the PDAF of the invention can be used in the treatment and diagnosis of the diseases associated with cell proliferation and immune response.

### Pharmaceutical Composition

The invention also provides a pharmaceutical composition comprising the polypeptides as shown in SEQ ID NO:1, 2 or 3, particularly for use in treating cell proliferation or inhibiting immune response. The pharmaceutical composition may contain suitable pharmaceutically-acceptable carriers, and optionally excipients and auxiliaries.

The pharmaceutical compositions of the invention may be manufactured in a manner that is known in the art, e.g., by means of conventional methods comprising the steps of mixing, dissolving, granulating, dragee-making, levigating, emulsifying, encapsulating, entrapping, and/or lyophilizing steps.

The pharmaceutical compositions of the invention may be administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual, or rectal means.

### Transgenic Animals

The invention also provides transgenic animals, which is introduced with a gene fragment containing the nucleic acid sequence of SEQ ID NO: 4, or a gene fragment containing the nucleic acid sequence of SEQ ID NO: 5, or a gene fragment containing the nucleic acid sequence of SEQ ID NO: 6. According to the invention, the transgenic animals may be any convenient animals, such as non-human mammal, for example as used in laboratory test procedures such as rodents, e.g., mice or rats; and for example as used for producing organs or tissues for transplantation, such as pigs and horses. The transgenic animals of the invention are conveniently obtained by introducing into animals the genes of the invention using conventional and convenient genetic manipulation techniques such as by microinjection or by infection with a recombinant vector. The gene may be directly or indirectly introduced into a cell or all the cells of an animal by introduction into a precursor cell. The genetic manipulation techniques include classical cross-breeding, in vitro fertilization, introduction of a recombinant DNA molecule, which may be integrated within a chromosome or may be extrachromosomally replicating DNA. The genes of the invention include the gene fragments containing the nucleic acid sequences of SEQ ID NOS: 4, 5, and 6, respectively.

According to the invention, the transgenic animals have non-specific immunosuppressive activity due to an introduction of the genes of the invention. Therefore, the transgenic animals may be used as organ or tissue sources for transplantation, which is more acceptable to humans.

The following Examples are offered by way of illustration and not by way of limitation.

### EXAMPLES

### Example 1 Production of PDAF

### Cloning of Human PDAF Gene

The placental tissues were grinded and mixed using a Glass grinder (Wheaton) in RNAZOL^{™}B solution (RNA isolation reagent). 0.1 ml of chloroform was added to the resulting solution and then the solution was placed on ice for 5 minutes. The resulting solution was centrifuged at 12,000×g at 4. for 15 minutes. Take 0.5 ml of the upper water layer and then add 0.5 ml of isopropanol to the layer. After completely mixing, the resulting solution was placed on the ice for 15 minutes and then centrifuged at 12,000×g at 4. for 15 minutes to precipitate RNA. 1ml of 75% alcohol was added to the precipitates to remove the salt in the RNA precipitates. The RNA precipitates were naturally dried in the air and then solved in appropriate amount of sterilized water. The OD₂₆₀ was determined for quantifying the RNA.

The reverse transcription reaction was performed using total RNA of placenta and oligo-d(T)₁₂₋₁₈ primers. The resulting first strand cDNA was bound to the complementary RNA to form a nucleic acid molecule. The nucleic acid molecule was treated with RNase H to obtain a primer for use in the synthesis of the second strand cDNA. Said cDNA was synthesized by using the primer and polymerase. The resulting cDNA was ligated into plasmid pCR3.1. The plasmid pCR3.1 was subsequently transformed into the host cells *E*. *Coli* DH5α. The host cells were transferred to the nitrocellulose membrane and lysed by using buffer containing strong basic and SDS to release the plasmid DNA. The plasmid DNA was treated with UV for fixing such DNA in the nitrocellulose membrane. The degenerated sequence of the peptide sequence GXXXXGKS of ATP-GTP (SEQ ID NO.7) binding site was used as the DNA probe to select the desired plasmid DNA of the host cells through hybridization reaction. The selected host cells were cultured and then the plasmid of the host cells was extracted. The DNA was sequenced through restriction map assay using various restriction enzymes or through automatic sequencing of double strand DNA using primer T7 and pCR3.1 reverse primer.

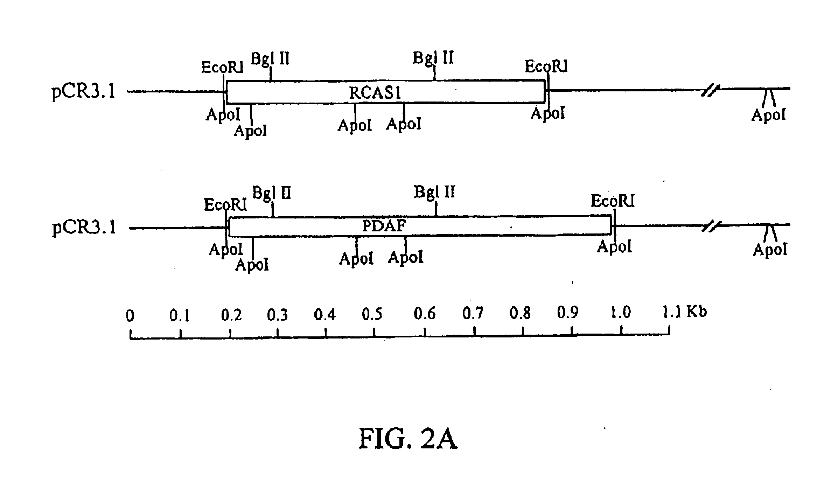

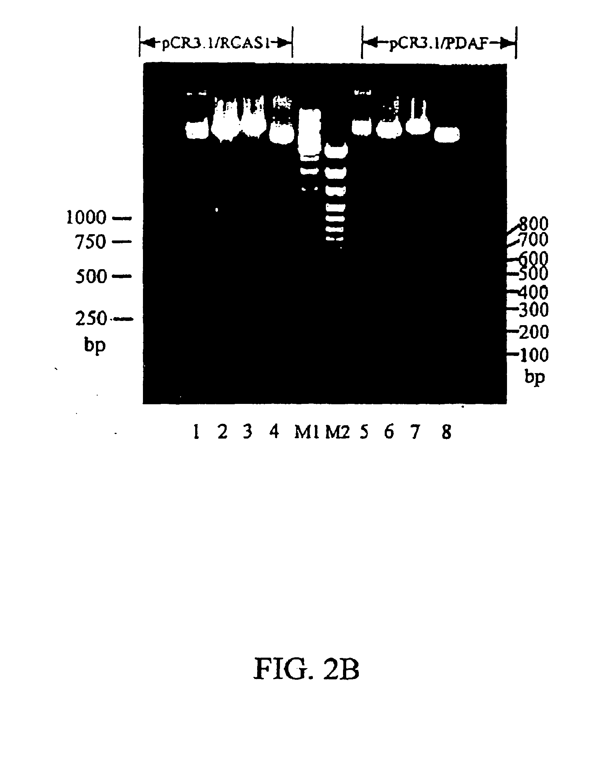

The sequenced DNA was analyzed by using DNASIS-Macv 2.0 software. After comparing with the sequences in Genebank, we have not found that any gene sequences are same as the sequence of the PDAF of the invention. However, it is found that the protein translation region of the novel PDAF gene is similar to that of RCAS1. As shown in Figs. 1A-1D, the protein translation region of the PDAF gene has 774 nucleic acids in length (SEQ ID NO: 4). Further, the restriction maps (Fig. 2A) of the PDAF and RCAS1 genes and the electrophoresis result of the restriction-enryme treated PDAF and RCAS1 genes (Fig. 2B) prove that the PDAF gene is indeed different from RCAS 1.

The protein translation region of the novel PDAF gene has additional 135 nucleic acids (SEQ ID NO: 5). The remaining 642 nucleic acids of the PDAF are completely same as that of RCAS1. In addition, it could not be found the sequences as the same as the above-mentioned 135 nucleic acids in the Genebank. Said sequence consisting of 135 nucleic acids is inserted between positions 522 and 523 of the sequence of RCAS1.

The sequence of 135 nucleic acids encodes a sequence consisting of 45 amino acids (SEQ ID NO. 2). After checking the Genebank, no similar sequences can be found. Only the sequence of amino acids 256-284 of the phosphatidyllinositol glycon of complementation class B (PIG-B) in the endoplasmic reticulum (ER) has low similarity to the sequence of 45 amino acids (see Fig. 3). Therefore, said sequence of 45 amino acids is a new sequence. Through the analysis of DNAISIS-Mac v.2.0 software, it is found that the sequence of 45 amino acids is a highly hydrophobic peptide (see Fig. 4). The amino acids 210-217 (GQWSYGKS) of said peptide are highly similar to the sequence ((A/G)XXXXGK(S/T)) (SEQ ID NO.8) of ATP-GTP binding site motif of Ras protein.

### Expression of PDAF in Tumor Cell Lines

Human mammary gland adenocarcinoma MCF-7, human colorectal adenocarcinoma HT29, human melanoma A375, human embryonal rhabdomyoma RD and human placenta cell were used to determine the expression of PDAF. The RNA was extracted and then the oligo-d(T)₁₂₋₁₈ were used as the primer for the reverse transcription reaction. The reaction products were subsequently performed PCR reaction using the sequences in the two ends of the protein translation region of PDAF gene as primers. The resulting reaction products were analyzed through agarose gel electrophoresis. As shown in Fig. 5, the above-mentioned tumor cells mainly express RCAS 1. Only RD cell lines express few amounts of PDAF. However, the placenta cell lines mainly express PDAF. On the contrary, few amounts of RCAS1 were expressed in the placenta cells.

### Expression and Purification of Fusion Protein

The RCAS1 gene fragments or PDAF gene fragments contained in pCR3.1 plasmid were cut by using restriction enzyme EcoRI and isolated with 1 % agarose gel. The isolated DNA fragments were purified by using Gene Clean III kit. The purified DNA was ligated to pGEX-3X vector (Amersham Pharmacia) and then the vector was transformed into *E*. *Coli.* The plasmids contained in the cells were extracted to obtain the plasmids pGEX-3X/RCAS1 and pGEX-3X/PDAF. The above two plasmids were transformed into *E. Coli* BL21 to express the fusion proteins.

The *E. Coli* cells containing the above two plasmids were cultured with shaking at 37. for overnight. The culture solution was inoculated to fresh AP/LB medium and cultured with shaking at 37. for 4 hours. 0.1mM of IPTG was added to the culture. Then, the culture was incubated with shaking at 25. for 4 hours to induce the expression of the fusion proteins. The cells were centrifuged at 8000×g at 4. for 15 minutes to collect the cells. The cells were suspended in 20ml of PBS buffer containing 1% Triton X-100. The cells were broken using sonicator. The insoluble precipitates were removed through centrifugation at 12,000×g at 4. for 15 minutes. The supernatant containing fusion protein was applied to affinity column with glutathione sepharose 4B (Amersham Pharmacia). The GST-fusion proteins were eluted with the buffer of pH8.0 containing 20mM reduced glutathione, 120mM NaCl, 50mM Tris-HCl. The eluted solution was dialyzed with PBS buffer at 4. and concentrated with Amicon® 8010. The concentration of the resulting proteins was determined using BCA protein assay reagent (Pierce).

The PCR was performed using primers designed from the extracellular protein regions of RCAS1 and PDAF to largely obtain the gene fragments encoding the extracellular protein regions of RCAS1 and PDAF. The gene fragments were treated with restriction enzyme BamHI and ligated to the vector pET32a(+) (Invitrogen) which was pretreated with BamHI. Then, the vector was transformed into *E*. *Coli* cells. The plasmids pET32a(+)/RCAS1.ECD and pET32a(+)/PDAF.ECD could be obtained through PCR, plasmid extraction and restriction map assay. The E. *Coli* BL21 (DE3) pLysS (Promega) were transformed with the above two plasmids.

The *E*. *Coli* cells containing the plasmids pET32a(+)/RCAS1.ECD and pET32a(+)/PDAF.ECD were incubated with shaking at 37. for overnight. The culture was inoculated to fresh AP/LB medium and subsequently incubated with shaking at 37. for 3 hours. 0.1mM of IPTG was added to the medium for inducing the large expression of the fusion protein. The culture was centrifuged at 8,000×g at 4. for 15 minutes to collect the cells. The cells were suspended in the buffer containing 50mM sodium phosphate, pH8.0, and 300mM sodium chloride and then broken by sonication. The insoluble precipitates were removed through centrifugation at 12,000×g at 4. for 15 minutes. The supernatant containing fusion protein was applied to an affinity column consisting of Ni-NTA SUPERFLOW (QIAGENE). The pET-fusion proteins were eluted with the buffer of pH8.0 containing 10% glycerol and 0-500mM imidazole in continuous gradients. The profile of fusion proteins pET-RCAS1.ECD, pET-PDAF.ECD, GST-RCAS1 and GST-PDAF was shown in Fig. 6. The eluted solution was dialyzed with PBS buffer at 4. and concentrated with AMICON® 8010 (ultrafiltration cell). The concentration of the resulting proteins was determined using BCA protein assay reagent (Pierce).

### Example 2. PI Staining and Flow Cytometry Assay

10⁸ of human peripheral blood mononuclear cell were incubated in RPMI medium with 10% bovine serum at 37. for 1-2 hours. The suspended cells were collected and then added to a 24-well culture dish coated with rabbit-anti-human immunoglobin IgM antibodies (CAPPEL). The culture dish was placed at room temperature for 1 hour for the attachment reaction. The lymphocytes attached to the rabbit-anti-human immunoglobin IgM antibodies were native B cells. The suspended cells were collected and 1 × 10⁶ cells/ml/well were added to a 24-well culture dish coated with mice-anti-human CD3 monoclonal antibodies (OKT3; 3µg/ml ; 1ml/well). The lymphocytes in the dish mainly were the active T cells. The fusion proteins prepared as described above were added to the 24-well culture dishes containing the above-mentioned B and T cells, and then the dishes were placed in a incubator and incubated with 5% CO₂ at 37. for 64-68 hours. The cells were centrifuged at 2400rpm for 5 minutes. The supernanent was removed and the hypotonic DNA staining buffer (3.5mM sodium citrate, 0.3% Triton X-100, pH 6.8) was added to the cells. 800µl of hypotonic DNA staining buffer and 30µg RNase A and 5µg propidium iodide were further added. The reaction was carried out for 30 minutes. The staining results were determined by flow cytometry. As shown in Fig. 7, the apoptotic rate of PBMC treated with GST-PDAF is higher than that of PBMC treated with GST-RCAS1.

Figures 8 and 9 show that the GST-PDAF can induce higher apoptosis in native B cell and activated T cell than GST-RCAS 1.

The PI staining and flow cytometry for human leukemia cell lines, Jurkat (T lymphoblast) and human erythroleukemia cell line, TF-1 (ATCC CRL-2003) were carried as described above. As shown in Fig. 10, the apoptotic rates of Jurkat cell treated with PBS buffer or GST protein (Control) were 3.95% and 5.22% respectively. The apoptotic rate of Jurkat cell treated with GST-RCAS1 was 7.9%, slightly higher than that of Jurkat cell treated with GST protein. However, the apoptotic rate of Jurket cell treated with GST-PDAF was largely higher than that of Jurkat cell treated with GST-RCAS1 and GST. Given the above, the PDAF of the invention can induce higher apoptosis in Jurkat cell than RCAS 1.

Erythroleukemia cell line TF-1 is a hemopoietic cell line with low differentiation. As shown in Fig. 11, the PDAF of the invention can induce higher apoptosis in TF-1 cell than RCAS1.

## Claims

1. A polypeptide, which comprises the amino acid sequence of SEQ ID NO: 1.

2. A polypeptide, which comprises the amino acid sequence of SEQ ID NO:2 or a sequence which is at least 70 % identical to SEQ ID NO:2 and has properties essentially identical to SEQ ID NO:2.

3. A polypeptide, which comprises the amino acid sequence of SEQ ID NO:3 or a sequence which is at least 70 % identical to SEQ ID NO:3 and has properties essentially identical to SEQ ID NO:3

4. The polypeptide as claimed in claim 1, for use in treating a cell proliferation disorder or inhibiting immune response.

5. The polypeptide as claimed in claim 2, for use in inhibiting the growth and differentiation of cells.

6. The polypeptide as claimed in claim 3, for use in treating cell proliferation disorder or inhibiting immune response.

7. The polypeptide as claimed in claim 1, for use in the treatment of cancer.

8. The polypeptide as claimed in claim 2, for use in the treatment of cancer.

9. The polypeptide as claimed in claim 3, for use in the treatment of cancer.

10. An isolated nucleic acid encoding the polypeptide of Claim 1 or the degenerate sequences thereof.

11. An isolated nucleic acid encoding the polypeptide of Claim 2 or the degenerate sequences thereof.

12. An isolated nucleic acid encoding the polypeptide of Claim 3 or the degenerate sequences thereof.

13. The isolated nucleic acid as claimed in claim 10, which is SEQ ID NO: 4.

14. The isolated nucleic acid as claimed in claim 11, which is SEQ ID NO: 5.

15. The isolated nucleic acid as claimed in claim 12, which is SEQ ID NO: 6.

16. An expression vector containing the nucleic acid sequences as claimed in claim 10.

17. An expression vector containing the nucleic acid sequences as claimed in claim 11.

18. An expression vector containing the nucleic acid sequences as claimed in claim 12.

19. A host cell containing the vector of claim 16.

20. A host cell containing the vector of claim 17.

21. A host cell containing the vector of claim 18.

22. A method for producing a polypeptide as claimed in claim 1, comprising the steps of:
a) culturing the host cell of claim 19 under conditions suitable for the expression of the polypeptide; and
b) recovering the polypeptide from the host cell culture.

23. A method for producing a polypeptide as claimed in claim 2, comprising the steps of:
a) culturing the host cell of claim 20 under conditions suitable for the expression of the polypeptide; and
b) recovering the polypeptide from the host cell culture.

24. A method for producing a polypeptide as claimed in claim 3, comprising the steps of:
a) culturing the host cell of claim 21 under conditions suitable for the expression of the polypeptide; and
b) recovering the polypeptide from the host cell culture.

25. A fusion protein comprising a polypeptide as claimed in Claim 1 and a foreign protein.

26. A fusion protein comprising a polypeptide as claimed in Claim 2 and a foreign protein.

27. A fusion protein comprising a polypeptide as claimed in Claim 3 and a foreign protein.

28. A pharmaceutical composition comprising the polypeptides as claimed in claim 1.

29. A pharmaceutical composition comprising the polypeptides as claimed in claim 2.

30. A pharmaceutical composition comprising the polypeptides as claimed in claim 3.

31. The pharmaceutical composition as claimed in claim 28, for use in treating cell proliferation disorder or inhibiting immune response.

32. The pharmaceutical composition as claimed in claim 29, for use in inhibiting the growth and differentiation of cells.

33. The pharmaceutical composition as claimed in claim 30, for use in treating cell proliferation disorder or inhibiting immune response.

34. A non-human transgenic animal, which is introduced with a gene fragment containing the nucleic acid sequence of SEQ ID NO: 4

35. A non-human transgenic animal, which is introduced with a gene fragment containing the nucleic acid sequence of SEQ ID NO: 5.

36. A non-human transgenic animal, which is introduced with a gene fragment containing the nucleic acid sequence of SEQ ID NO: 6.

## Patentansprüche

1. Polypeptid, die Aminosäuresequenz von SEQ ID NO: 1 umfassend.

2. Polypeptid, die Aminosäuresequenz von SEQ ID NO:2 oder einer Sequenz umfassend, die zu 70% zu SEQ ID NO:2 identisch ist und im Wesentlichen gleiche Eigenschaften wie SEQ ID NO:2 aufweist.

3. Polypeptid, die Aminosäuresequenz von SEQ ID NO:3 oder einer Sequenz umfassend, die zu 70% zu SEQ ID NO:3 identisch ist und im Wesentlichen gleiche Eigenschaften wie SEQ ID NO:2 aufweist.

4. Polypeptid nach Anspruch 1 für eine Verwendung bei einer Behandlung einer Zellproliferationserkrankung oder zu einem Inhibieren einer Immunreaktion.

5. Polypeptid nach Anspruch 2 für eine Verwendung bei einem Inhibieren des Wachstums und Differenzierens von Zellen.

6. Polypeptid nach Anspruch 3 für eine Verwendung bei einer Behandlung einer Zellproliferationserkrankung oder zu einem Inhibieren einer Immunreaktion.

7. Polypeptid nach Anspruch 1 für eine Verwendung bei der Behandlung von Krebs.

8. Polypeptid nach Anspruch 2 für eine Verwendung bei der Behandlung von Krebs.

9. Polypeptid nach Anspruch 3 für eine Verwendung bei der Behandlung von Krebs.

10. Isolierte Nukleinsäure, welche das Polypeptid nach Anspruch 1 oder die degenerierten Sequenzen davon codiert.

11. Isolierte Nukleinsäure, welche das Polypeptid nach Anspruch 2 oder die degenerierten Sequenzen davon codiert.

12. Isolierte Nukleinsäure, welche das Polypeptid nach Anspruch 3 oder die degenerierten Sequenzen davon codiert.

13. Isolierte Nukleinsäure gemäß Anspruch 10, welche SEQ ID NO: 4 ist.

14. Isolierte Nukleinsäure gemäß Anspruch 11, welche SEQ ID NO: 5 ist.

15. Isolierte Nukleinsäure gemäß Anspruch 12, welche SEQ ID NO: 6 ist.

16. Expressionsvektor die Nukleinsäuresequenzen gemäß Anspruch 10 umfassend.

17. Expressionsvektor die Nukleinsäuresequenzen gemäß Anspruch 11 umfassend.

18. Expressionsvektor die Nukleinsäuresequenzen gemäß Anspruch 12 umfassend.

19. Wirtszelle den Vektor gemäß Anspruch 16 umfassend.

20. Wirtszelle den Vektor gemäß Anspruch 17 umfassend.

21. Wirtszelle den Vektor gemäß Anspruch 18 umfassend.

22. Verfahren zur Herstellung eines Polypeptids gemäß Anspruch 1, umfassend die Schritte von:
a) Kultivieren der Wirtszelle gemäß Anspruch 19 bei Bedingungen, welche für die Expression des Polypeptids geeignet sind; und
b) Erhalten des Polypeptids von der Wirtszellkultur.

23. Verfahren zur Herstellung eines Polypeptids gemäß Anspruch 2, umfassend die Schritte von:
a) Kultivieren der Wirtszelle gemäß Anspruch 20 bei Bedingungen, welche für die Expression des Polypeptids geeignet sind; und
b) Erhalten des Polypeptids von der Wirtszellkultur.

24. Verfahren zur Herstellung eines Polypeptids gemäß Anspruch 3, umfassend die Schritte von:
a) Kultivieren der Wirtszelle gemäß Anspruch 21 bei Bedingungen, welche für die Expression des Polypeptids geeignet sind; und
b) Erhalten des Polypeptids von der Wirtszellkultur.

25. Fusionsprotein ein Polypeptid gemäß Anspruch 1 und ein Fremdprotein umfassend.

26. Fusionsprotein ein Polypeptid gemäß Anspruch 2 und ein Fremdprotein umfassend.

27. Fusionsprotein ein Polypeptid gemäß Anspruch 3 und ein Fremdprotein umfassend.

28. Pharmazeutische Zusammensetzung die Polypeptide gemäß Anspruch 1 umfassend.

29. Pharmazeutische Zusammensetzung die Polypeptide gemäß Anspruch 2 umfassend.

30. Pharmazeutische Zusammensetzung die Polypeptide gemäß Anspruch 3 umfassend.

31. Pharmazeutische Zusammensetzung gemäß Anspruch 28 für eine Verwendung bei einer Behandlung einer Zellproliferationserkrankung oder zu einem Inhibieren einer Immunreaktion.

32. Pharmazeutische Zusammensetzung gemäß Anspruch 29 für eine Verwendung bei einem Inhibieren des Wachstums und Differenzierens von Zellen.

33. Pharmazeutische Zusammensetzung gemäß Anspruch 30 für eine Verwendung bei einer Behandlung einer Zellproliferationserkrankung oder zu einem Inhibieren einer Immunreaktion.

34. Nicht-menschliches transgenes Tier, in das ein Genfragment eingebracht wurde, das die Nukleinsäuresequenz gemäß SEQ ID NO: 4 umfasst.

35. Nicht-menschliches transgenes Tier, in das ein Genfragment eingebracht wurde, das die Nukleinsäuresequenz gemäß SEQ ID NO: 5 umfasst.

36. Nicht-menschliches transgenes Tier, in das ein Genfragment eingebracht wurde, das die Nukleinsäuresequenz gemäß SEQ ID NO: 6 umfasst.

## Revendications

1. Un polypeptide qui comprend la séquence d'acides aminés de SEQ ID NO : 1.

2. Un polypeptide qui comprend la séquence d'acides aminés de SEQ ID NO : 2 ou une séquence qui est identique à au moins 70 % à la séquence SEQ ID NO : 2 et qui a des propriétés essentiellement identiques à celles de SEQ ID NO : 2.

3. Un polypeptide qui comprend la séquence d'acides aminés de SEQ ID NO : 3 ou une séquence qui est identique à au moins 70 % à la séquence SEQ ID NO : 3 et qui a des propriétés essentiellement identiques à celles de SEQ ID NO : 3.

4. Le polypeptide tel que revendiqué dans la revendication 1 pour utilisation dans le traitement d'un désordre de prolifération cellulaire ou l'inhibition de la réponse immunitaire.

5. Le polypeptide tel que revendiqué dans la revendication 2 pour utilisation dans l'inhibition de la croissance et de la différentiation de cellules.

6. Le polypeptide tel que revendiqué dans la revendication 3 pour utilisation dans le traitement du désordre de prolifération cellulaire ou l'inhibition de la réponse immunitaire.

7. Le polypeptide tel que revendiqué dans la revendication 1 pour utilisation dans le traitement du cancer.

8. Le polypeptide tel que revendiqué dans la revendication 2 pour utilisation dans le traitement du cancer.

9. Le polypeptide tel que revendiqué dans la revendication 3 pour utilisation dans le traitement du cancer.

10. Un acide nucléique isolé codant le polypeptide de la revendication 1 ou les séquences dégénérées de celui-ci.

11. Un acide nucléique isolé codant le polypeptide de la revendication 2 ou les séquences dégénérées de celui-ci.

12. Un acide nucléique isolé codant le polypeptide de la revendication 3 ou les séquences dégénérées de celui-ci.

13. L'acide nucléique tel que revendiqué dans la revendication 10 qui est SEQ ID NO : 4.

14. L'acide nucléique tel que revendiqué dans la revendication 11 qui est SEQ ID NO : 5.

15. L'acide nucléique tel que revendiqué dans la revendication 12 qui est SEQ ID NO : 6.

16. Un vecteur d'expression contenant les séquences d'acides aminés telles que revendiquées dans la revendication 10.

17. Un vecteur d'expression contenant les séquences d'acides aminés telles que revendiquées dans la revendication 11.

18. Un vecteur d'expression contenant les séquences d'acides aminés telles que revendiquées dans la revendication 12.

19. Une cellule hôte contenant le vecteur de la revendication 16.

20. Une cellule hôte contenant le vecteur de la revendication 17.

21. Une cellule hôte contenant le vecteur de la revendication 18.

22. Une méthode pour produire un polypeptide tel que revendiqué dans la revendication 1, comprenant les étapes de :
a) cultiver la cellule hôte de la revendication 19 sous des conditions appropriées à l'expression du polypeptide ; et
b) récupérer le polypeptide à partir de la culture de cellule hôte.

23. Une méthode pour produire un polypeptide tel que revendiqué dans la revendication 2, comprenant les étapes de :
a) cultiver la cellule hôte de la revendication 20 sous des conditions appropriées à l'expression du polypeptide ; et
b) récupérer le polypeptide à partir de la culture de cellule hôte.

24. Une méthode pour produire un polypeptide tel que revendiqué dans la revendication 3, comprenant les étapes de :
a) cultiver la cellule hôte de la revendication 21 sous des conditions appropriées à l'expression du polypeptide ; et
b) récupérer le polypeptide à partir de la culture de cellule hôte.

25. Une protéine de fusion comprenant un polypeptide tel que revendiqué dans la revendication 1 et une protéine étrangère.

26. Une protéine de fusion comprenant un polypeptide tel que revendiqué dans la revendication 2 et une protéine étrangère.

27. Une protéine de fusion comprenant un polypeptide tel que revendiqué dans la revendication 3 et une protéine étrangère.

28. Une composition pharmaceutique comprenant les polypeptides tels que revendiqués dans la revendication 1.

29. Une composition pharmaceutique comprenant les polypeptides tels que revendiqués dans la revendication 2.

30. Une composition pharmaceutique comprenant les polypeptides tels que revendiqués dans la revendication 3.

31. La composition pharmaceutique telle que revendiquée dans la revendication 28 pour utilisation dans le traitement du désordre de prolifération cellulaire ou l'inhibition de la réponse immunitaire.

32. La composition telle que revendiquée dans la revendication 29 pour utilisation dans l'inhibition de la croissance et de la différentiation de cellules.

33. La composition pharmaceutique de la revendication 30 pour utilisation dans le traitement du désordre de prolifération cellulaire ou l'inhibition de la réponse immunitaire.

34. Un animal transgénique non humain dans lequel est introduit un fragment de gène contenant la séquence d'acides nucléiques de SEQ ID NO : 4.

35. Un animal transgénique non humain dans lequel est introduit un fragment de gène contenant la séquence d'acides nucléiques de SEQ ID NO : 5.

36. Un animal transgénique non humain dans lequel est introduit un fragment de gène contenant la séquence d'acides nucléiques de SEQ ID NO : 6.
